# EUROPEAN PATENT APPLICATION

(11) **EP 2 412 352 A1**
(43) Date of publication of application: **01.02.2012**
(21) Application number: 10756217.5
(22) Date of filing: 26.03.2010
(51) Int. Cl.: A61F 13/49, A61F 13/514

(54) **ABSORPTIVE ARTICLE**

(30) Priority: 26.03.2009 JP 2009076909
(71) Applicant: Unicharm Corporation, Ehime 799-0111 (JP)
(72) Inventor: OKU, Tomomi, Kanonji-shi Kagawa 769-1602 (JP); SAKAGUCHI, Satoru, Kanonji-shi Kagawa 769-1602 (JP)
(74) Representative: Vaughan, Jennifer Ann
(86) International application number: PCT/JP2010/055362
(87) International publication number: WO 2010/110421

(57) **Abstract**

An absorbent article includes: a belly waistline portion to be fitted to a belly waistline of a wearer; a rear waistline portion to be fitted to a rear waistline of the wearer; a crotch portion to be fitted to a crotch of the wearer; a central portion in a width direction of the absorbent article. The central portion includes an absorber; and a lateral portion located outside the central portion in the width direction of the absorbent article. The backsheet includes a stretched portion stretched by stretch processing in the width direction of the absorbent article perpendicular to a belly-to-rear direction extending from the belly waistline portion to the rear waistline portion, and a unstretched portion not stretched by the stretch processing. The stretched portion includes a central stretched portion located in at least part of the central portion within the crotch portion, and a lateral stretched portion located in at least part of the lateral portion within the crotch portion.

## Description

### [Technical Field]

The present invention relates to an absorbent article including a liquid-permeable topsheet, a liquid-impermeable backsheet, and an absorber provided between the topsheet and the backsheet.

### [Background Art]

Conventionally, an absorbent article such as a pants-type diaper, a sanitary napkin or a panty liner includes a belly waistline portion to be fitted to the belly (belly waistline) of a wearer, a rear waistline portion to be fitted to the back (rear waistline) of the wearer, and a crotch portion to be fitted to the crotch of the wearer.

The crotch portion is provided with an absorber configured to absorb body fluids discharged from the wearer and a leak proof portion configured to prevent leakage of the body fluids of the wearer from the absorbent article. The leak proof portion is provided to extend in a belly-to-rear direction (i.e., longitudinal direction) from the belly waistline portion to the rear waistline portion, and is folded to form multiple layers at edge portions on the both sides of the absorber.

A stretchable cord-shaped member such as a rubber is provided in a stretched state inside the leak proof portion. The leak proof portion rises in a direction away from the absorber because the stretched cord-shaped member tries to return to the unstretched state. With this force, the absorbent article curls up along a line from the wearer's belly to the wearer's back. Accordingly, the leak proof portion comes in contact with the skin of the wearer, and a space (i.e., pocket) is formed between the crotch of the wearer and the absorber. Thus, it is possible to prevent side leakage, i.e., to prevent body fluids of the wearer from leaking out from both edge portions of the absorber (Patent Document 1, for example).

However, the above-mentioned conventional absorbent article has the following problem. Specifically, in order to form a pocket, the leak proof portion needs to be folded in multiple layers at both edge portions of the absorber. This causes a problem that a wearer may feel a sense of discomfort when wearing the absorbent article because the leak proof portion folded in multiple layers comes in contact with the skin of the wearer.

### [Prior Art Document]

### [Patent Document]

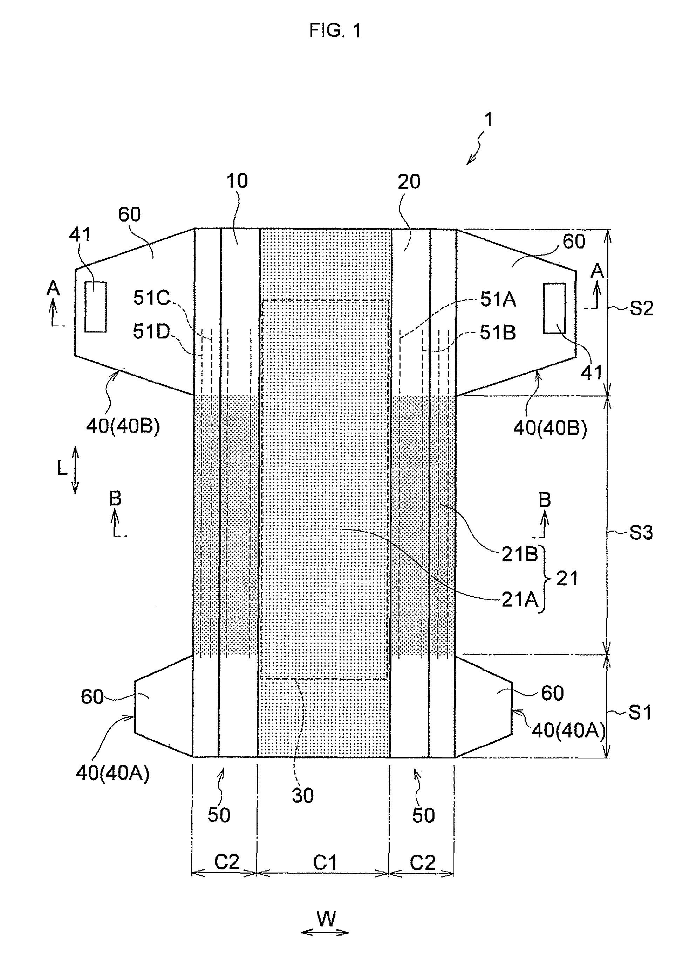

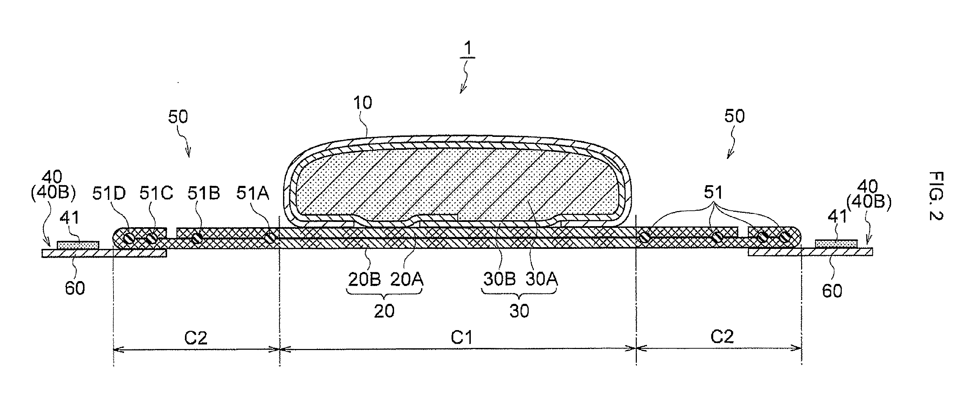

[Patent Document 1] Japanese Patent Application Publication No. Showa 63-235501 (pp. 1 and 2, Figs. 1 and 2)

### [Summary of Invention]

An absorbent article includes: a liquid-permeable topsheet; a liquid-impermeable backsheet; an absorber provided between the topsheet and the backsheet; a belly waistline portion to be fitted to a belly waistline of a wearer; a rear waistline portion to be fitted to a rear waistline of the wearer; a crotch portion to be fitted to a crotch of the wearer; a central portion in a width direction of the absorbent article. The central portion includes the absorber; and a lateral portion located outside the central portion in the width direction of the absorbent article. The backsheet includes a stretched portion stretched by stretch processing in the width direction of the absorbent article perpendicular to a belly-to-rear direction extending from the belly waistline portion to the rear waistline portion, and a unstretched portion not stretched by the stretch processing. The stretched portion includes a central stretched portion located in at least part of the central portion within the crotch portion, and a lateral stretched portion located in at least part of the lateral portion within the crotch portion.

### [Brief Description of Drawings]

[Fig. 1]
   Fig. 1 is a plan view showing an absorbent article 1 according to a first embodiment.
[Fig. 2]
   Fig. 2 is a cross-sectional view (cross-sectional view taken along line A-A of Fig. 1) showing the absorbent article 1 according to the first embodiment.
[Fig. 3]

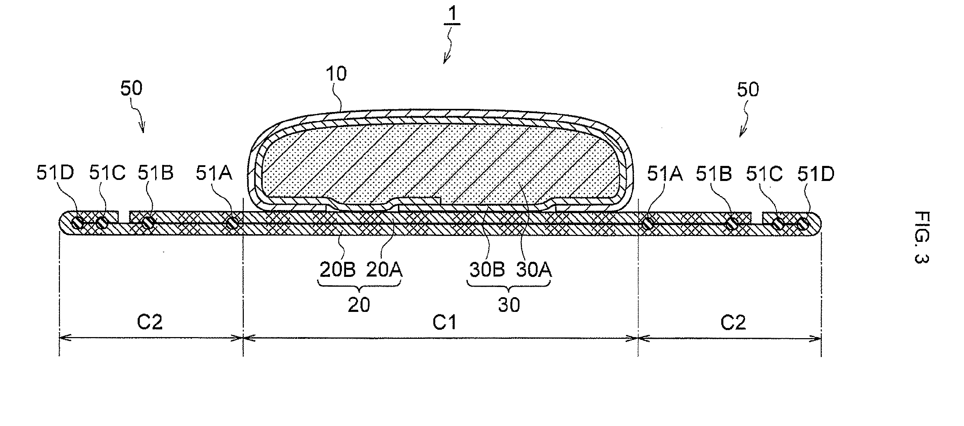

   Fig. 3 is a cross-sectional view (cross-sectional view taken along line B-B of Fig. 1) showing the absorbent article 1 according to the first embodiment.
[Fig. 4]

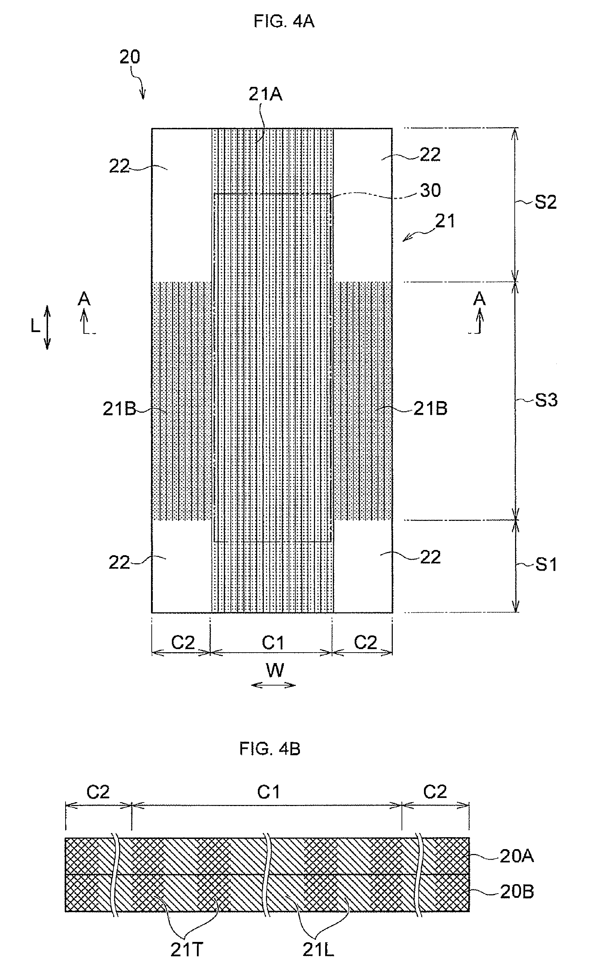

   Fig. 4 (a) is a plan view showing a backsheet 20 according to the first embodiment, and Fig. 4 (b) is an enlarged cross-sectional view showing a backsheet 20 according to the first embodiment.
[Fig. 5]

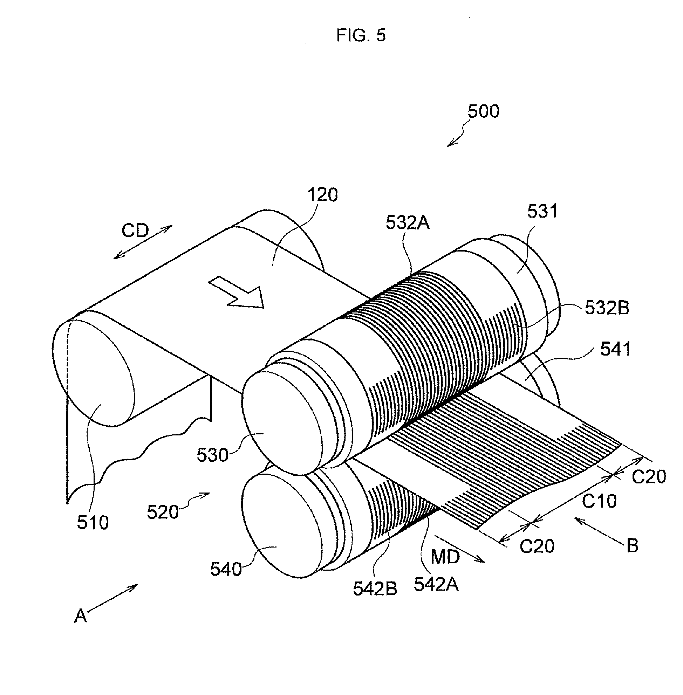

   Fig. 5 is a perspective view showing a stretch processor 500 according to the first embodiment.
[Fig. 6]

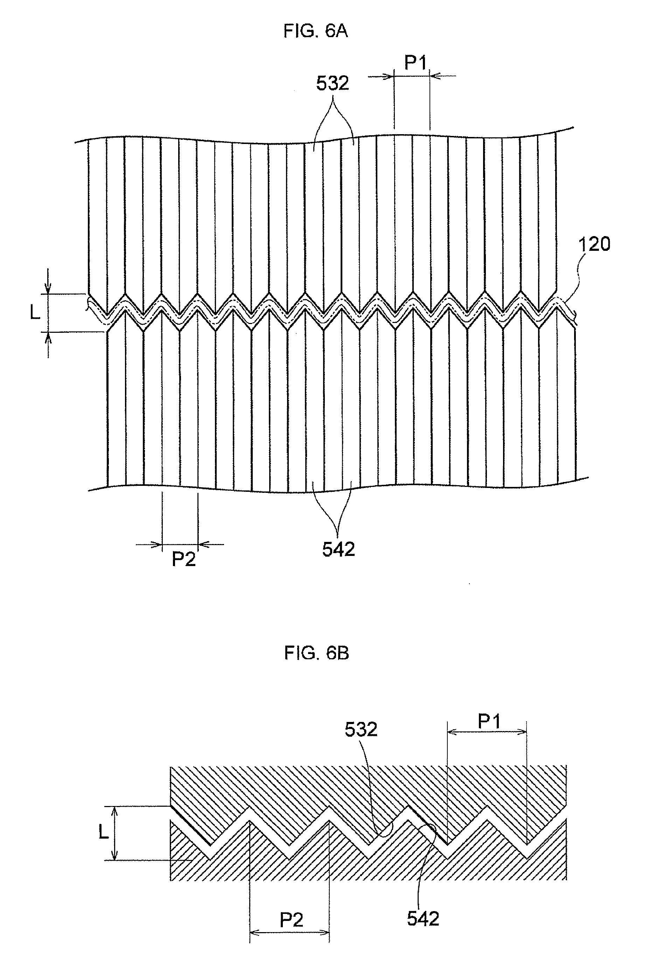

   Figs. 6 (a) and 6 (b) are respectively a front view and an enlarged front view showing the stretch processor 500 according to the first embodiment.
[Fig. 7]

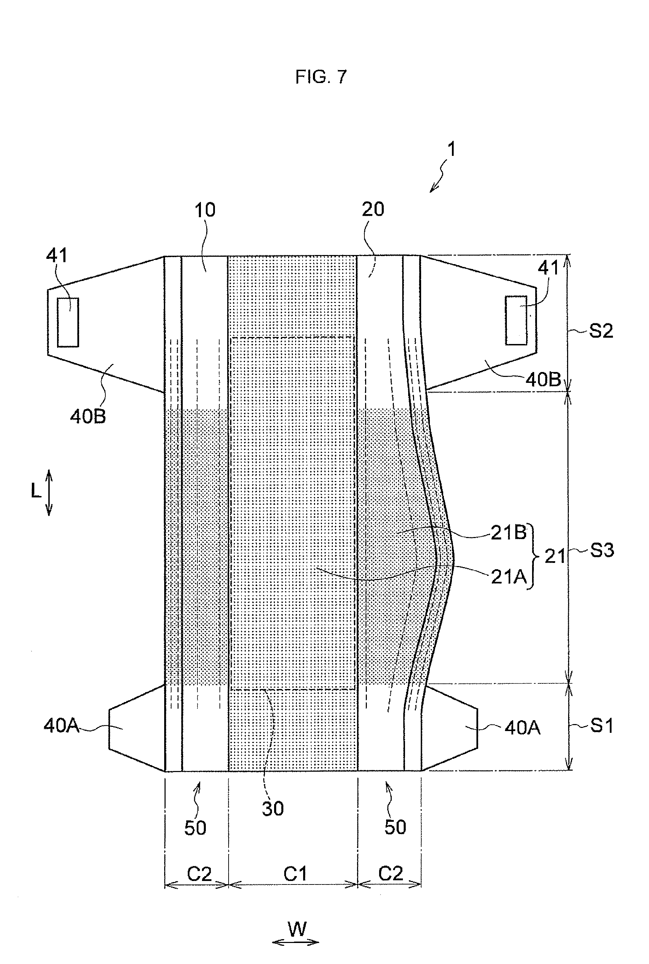

   Fig. 7 is a plan view showing the absorbent article 1 for explaining the advantageous effects.
[Fig. 8]

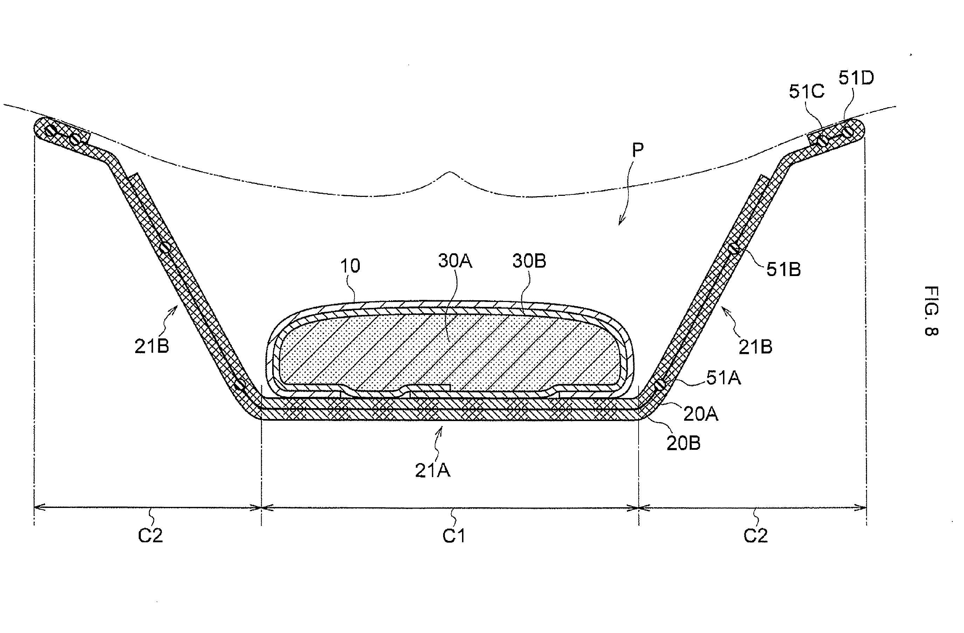

   Fig. 8 is a cross-sectional view showing the absorbent article 1 for explaining the advantageous effects (when a wearer wears the absorbent article 1).
[Fig. 9]

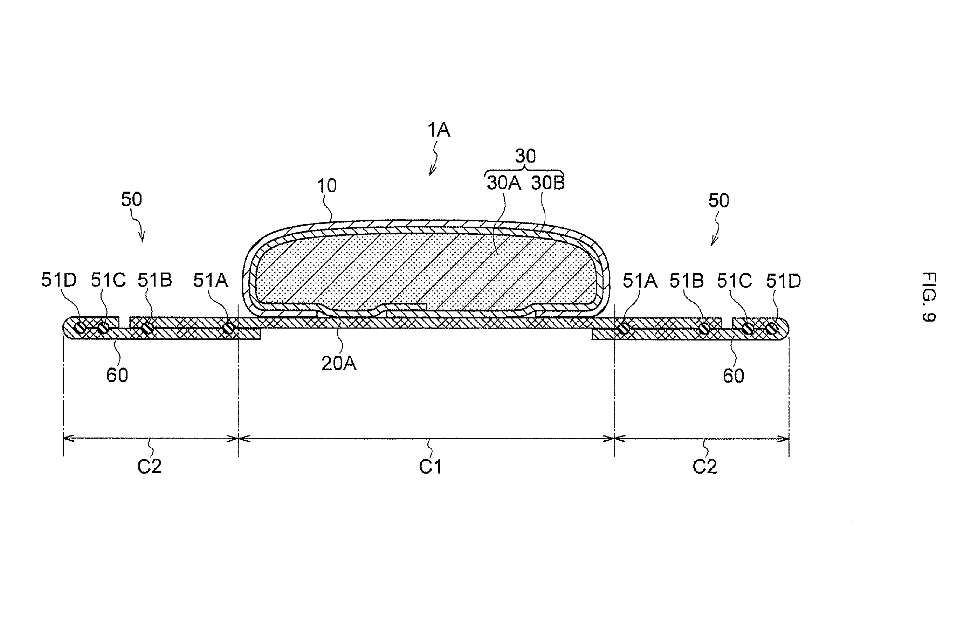

   Fig. 9 is a cross-sectional view showing a part of an absorbent article 1A according to a modified example 1.
[Fig. 10]

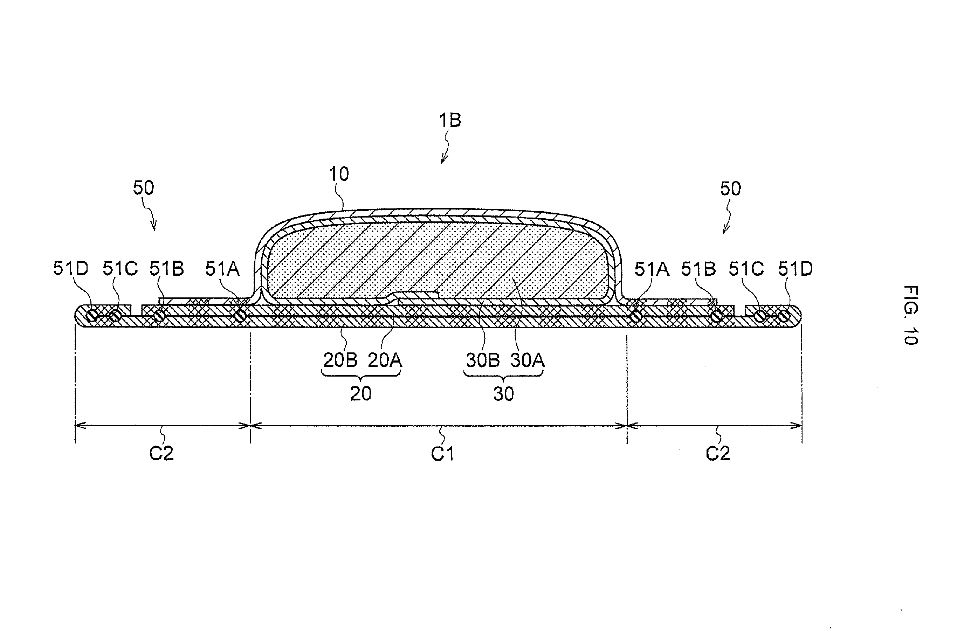

   Fig. 10 is a cross-sectional view showing a part of an absorbent article 1B according to a modified example 2.
[Fig. 11]

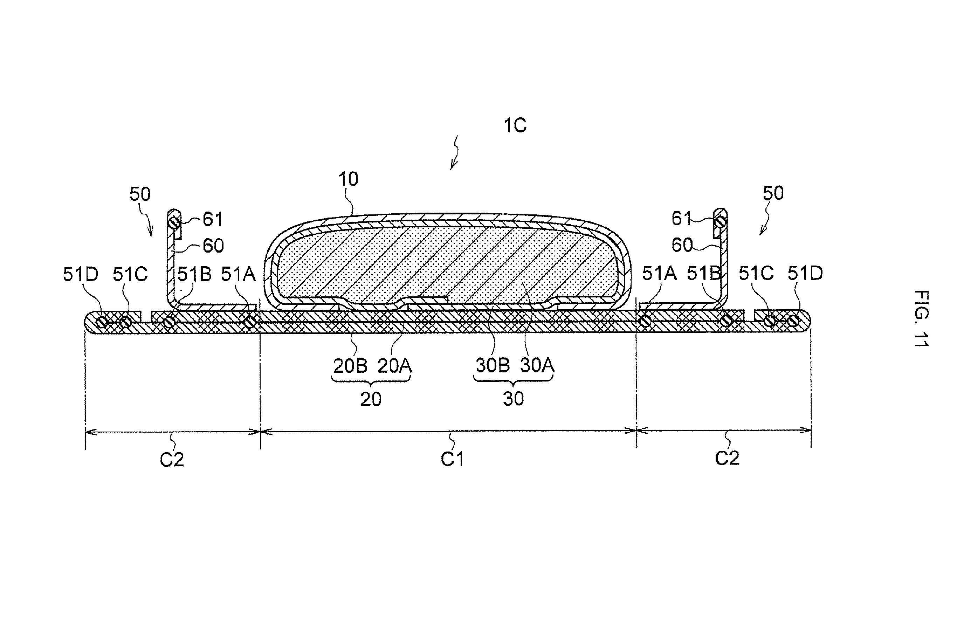

   Fig. 11 is a cross-sectional view showing a part of an absorbent article 1C according to a modified example 3.
[Fig. 12]

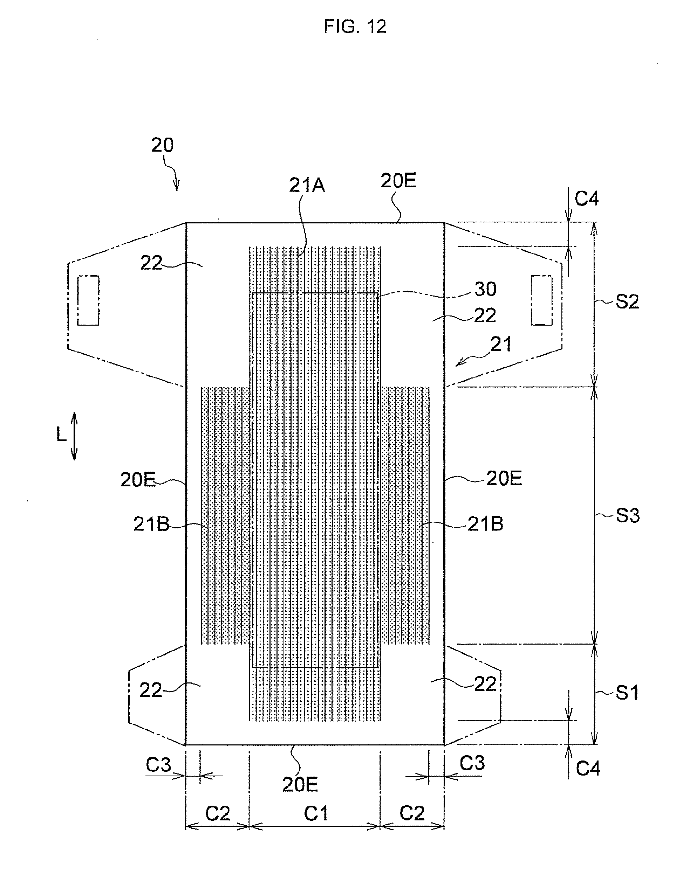

   Fig. 12 is a plan view showing a backsheet 20 according to a second embodiment.

### [Description of Embodiments]

Hereinafter, an absorbent article according to the embodiments will be described with reference to the drawings. Specifically, a first embodiment, a second embodiment, a third embodiment, and other embodiments will be described.

Note that, in the following description of the drawings, the same or similar reference signs denote the same or similar portions. In addition, it should be noted that the drawings are schematic and ratios of dimensions and the like are different from actual ones.

Therefore, specific dimensions and the like should be determined in consideration of the following description. Moreover, it is a matter of course that the drawings include portions having different dimensional relationships and ratios from each other.

### [First embodiment]

In the first place, a configuration of an absorbent article 1 according to a first embodiment will be described with reference to the drawings. Fig. 1 is a plan view showing the absorbent article 1 according to the first embodiment. Fig. 2 is a cross-sectional view (cross-sectional view taken along line A-A of Fig. 1) showing the absorbent article 1 according to the first embodiment. Fig. 3 is a cross-sectional view (cross-sectional view taken along line B-B of Fig. 1) showing the absorbent article 1 according to the first embodiment. Note that, in the first embodiment, the absorbent article 1 is an open-type diaper.

As shown in Figs. 1 to 3, the absorbent article 1 has a vertically long shape in a belly-to-rear direction (hereinafter, called a longitudinal direction L of the absorbent article 1) corresponding to a direction extending from the belly waistline (belly) to the rear waistline (back) of a wearer.

In the longitudinal direction L of the absorbent article 1, the absorbent article 1 includes: a belly waistline portion S1 that corresponds to the belly waistline of the wearer; a rear waistline portion S2 to be fitted to the rear waistline of the wearer; and a crotch portion S3 to be fitted to the crotch of the wearer and is located between the belly waistline portion S1 and the rear waistline portion S2.

Additionally, in a width direction W perpendicular to the longitudinal direction L of the absorbent article 1, the absorbent article 1 includes: a central portion C1 having an absorber 30, which will be described later, in the width direction W of the absorbent article 1; and lateral portions C2 each located outside the central portion C1 in the width direction W of the absorbent article 1.

The absorbent article 1 includes a topsheet 10, a backsheet 20 and the absorber 30. In addition, the absorbent article 1 is provided with waist flap portions 40 and side flap portions 50.

The topsheet 10 is provided on a side to come in contact with the skin of the wearer. Both end portions of the topsheet 10 in the width direction W are wrapped around the absorber 30. The topsheet 10 is formed of a liquid-permeable sheet such as a hydrophilic nonwoven or woven fabric, a perforated plastic film, or a perforated hydrophobic nonwoven fabric.

The backsheet 20 is provided on a side opposite to the topsheet 10, being the side not to come into contact with the wearer. The backsheet 20 is formed of a liquid-impermeable backsheet 20A such as a waterproof film (a polyethylene film, for example), and an exterior sheet 20B such as a nonwoven fabric attached to a surface of the backsheet 20A on a side opposite to the side where the absorber 30 is provided. The backsheet 20 does not always need to be formed of the backsheet 20A and the exterior sheet 20B. Instead, the backsheet 20 may be formed of only the backsheet 20A. A detailed description of the backsheet 20 will be given later.

The absorber 30 is provided between the topsheet 10 and the backsheet 20. The absorber 30 is formed of a mixed powder 30A including a ground pulp, a highly water-absorbent polymer or the like, and a coating material 30B such as a tissue covering the mixed powder 30A.

Here, the topsheet 10, the backsheet 20 and the absorber 30 are joined to each other by an adhesive (for example, hot melt adhesive) or by heat sealing.

The waist flap portions 40 are provided in the belly waistline portion S1 and the rear waistline portion S2 in the longitudinal direction L of the absorbent article 1. The waist flap portions 40 include a pair of front flap portions 40A located in the belly waistline portion S1 and a pair of back flap portions 40B located in the rear waistline portion S2.

The front flap portions 40A are formed with side sheets 60 extended toward the outer side of the absorbent article 1 in the width direction W. Instead of the side sheets 60 joined with the exterior sheet 20B of the backsheet 20, here, the backsheet 20 (the backsheet 20A and the exterior sheet 20B) may be extended to form the front flap portions 40A.

In the same manner as the front flap portions 40A, the back flap portions 40B are formed with side sheets 60 extended toward the outer side of the absorbent article 1 in the width direction W. Each of the back flap portions 40B is provided with an engaging portion configured to engage with the belly waistline portion S1. The engaging portion 41 is provided on a surface of the back flap portion 40B on the side where the absorber 30 is provided, and is arranged on either of the outer sides of the absorbent article 1 in the width direction W. The engaging portion 41 is formed of a hook-and-loop fastener, an adhesive tape, or the like.

In the case where the engaging portion 41 is a hook-and-loop fastener (hook member), the belly waistline portion S1 may be provided with an engaged portion (loop member) in a portion to be engaged with the engaging portion 41, or may itself serve as the engaged portion if the belly waistline portion S1 is formed of a nonwoven fabric.

Each of the side flap portions 50 is formed by extending the backsheet 20 (the backsheet 20A and the exterior sheet 20B). Each side flap portion 50 is provided outside the absorber 30 in the width direction W of the absorbent article 1 and extends in the longitudinal direction L of the absorbent article 1. The side flap portion 50 is provided with a cord-shaped member 51 such as a rubber (for example, a polyurethane rubber) that is stretchable in the longitudinal direction L of the absorbent article 1. The cord-shaped member 51 is provided to extend in the longitudinal direction L of the absorbent article 1 while being stretched in the longitudinal direction L of the absorbent article 1.

The cord-shaped member 51 includes a first cord-shaped member 51A, a second cord-shaped member 51B, a third cord-shaped member 51C and a fourth cord-shaped member 51D from the inner side to the outer side of the absorbent article 1 in the width direction W. The first cord-shaped member 51A and the second cord-shaped member 51B are provided between the backsheet 20A and the exterior sheet 20B. The third cord-shaped member 51C and the fourth cord-shaped member 51D are sandwiched by the doubled exterior sheet 20B that extends outwardly from the backsheet 20A in the width direction W of the absorbent article 1 and then is folded back into two.

Next, the above-mentioned backsheet 20 will be described with reference to the drawings. Fig. 4 (a) is a plan view showing the backsheet 20 according to the first embodiment, and Fig. 4 (b) is an enlarged cross-sectional view showing the backsheet 20 according to the first embodiment.

As shown in Fig. 4 (a), in the width direction W of the absorbent article 1, the backsheet 20 (the backsheet 20A and the exterior sheet 20B) includes a stretched portion 21 stretched by stretch processing, and unstretched portions 22 not stretched by stretch processing.

The stretched portion 21 has a basis weight (mass per unit area) lower than the unstretched portions 22 because the stretched portion 21 is stretched by the stretch processing. As shown in Fig. 4 (b), the stretched portion 21 includes a sparse portion 21L with a large amount of stretch and a dense portion 21T with a small amount of stretch, formed in an alternate manner.

The stretched portion 21 includes a central stretched portion 21A located in the central portion C1 inside the crotch portion S3, and lateral stretched portions 21B located in the respective lateral portions C2 within the crotch portion S3. In other words, the stretched portion 21 is provided in the entire crotch portion S3 in the width direction W of the absorbent article 1, and is provided in the entire central portion C1 in the longitudinal direction L of the absorbent article 1.

The stretched portion 21 is stretched to at most 2.5 times the original length of the backsheet 20 before the stretch processing. The stretch rate of the lateral stretched portions 21B is larger than the stretch rate of the central stretched portion 21A. In addition, the stretched portion 21 preferably has such transparency that the inside of the absorbent article 1 can be seen from the outside.

The unstretched portions 22 are portions excluding the stretched portion 21. Specifically, the unstretched portions 22 are provided in at least part of the belly waistline portion S1 and the rear waistline portion S2. In other words, the unstretched portions 22 are provided in a pair of the lateral portions C2 within the belly waistline portion S1 and in a pair of the lateral portions C2 within the rear waistline portion S2.

Next, a stretch processor 500 configured to form the stretched portion 21 in the backsheet 20 will be described with reference to the drawings. Fig. 5 is a perspective view showing the stretch processor 500 according to the first embodiment. Fig. 6 (a) is a plan view (view indicated by arrow A in Fig. 5) showing the stretch processor 500 according to the first embodiment, and Fig. 6 (b) is an enlarged front view showing the stretch processor 500 according to the first embodiment.

As shown in Figs. 5 to 6(b), the stretch processor 500 stretches a backsheet continuum 120 in a width direction CD perpendicular to a conveyance direction MD. Here, the backsheet continuum 120 is long and includes the backsheets 20 continuously. The stretch processor 500 includes a preheating roll 510 and a stretch processing mechanism 520.

The preheating roll 510 is provided upstream of the stretch processing mechanism 520 in the conveyance direction MD of the backsheet continuum 120. The preheating roll 510 heats the backsheet continuum 120 before the backsheet continuum 120 reaches the stretch processing mechanism 520. The preheating roll 510 has a predetermined temperature (for example, 100°C).

The stretch processing mechanism 520 further heats and also stretches the backsheet continuum 120 having passed through the preheating roll 510. The stretch processing mechanism 520 has a predetermined temperature (for example, 50°C to 80°C). The stretch processing mechanism 520 includes an upper roll 530 and a lower roll 540.

The upper roll 530 is provided on the upper side of the backsheet continuum 120. The upper roll 530 includes an upper roll body 531 configured to rotate about the axle, and multiple upper convex portions 532 configured to press the backsheet continuum 120.

Each of the upper convex portions 532 is provided to extend in a rotational direction of the upper roll body 531, and protrudes from the outer circumference of the upper roll body 531. The multiple upper convex portions 532 are arranged side by side in an axial direction of the upper roll body 531. Each of the upper convex portions 532 has a tapered shape (in the drawings, approximately a triangular shape) in a cross section when taken in the axial direction of the upper roll body 531. The upper convex portions 532 include multiple central convex portions 532A, and multiple lateral convex portions 532B.

The central convex portions 532A form the central stretched portion 21A by pressing a central portion C10 of the backsheet continuum 120 to be processed into the central portion C1 of the absorbent article 1. The central convex portions 532A are provided over the entire circumference of the upper roll body 531 in a circumferential direction of the upper roll body 531.

The lateral convex portions 532B form the lateral stretched portions 21B by pressing lateral portions C20 of the backsheet continuum 120 to be processed into the lateral portions C2 of the absorbent article 1. The lateral convex portions 532B are provided discontinuously on the outer circumference of the upper roll body 531. Note that, the height of the lateral convex portions 532B is higher than that of the central convex portions 532A.

The lower roll 540 is provided on the side opposite to the upper roll 530 across the backsheet continuum 120. The lower roll 540 includes a lower roll body 541 configured to rotate about the axle, and multiple lower convex portions 542 configured to fit with the multiple upper convex portions 532.

Each of the lower convex portions 542 is provided to extend in a rotational direction of the lower roll body 541, and protrudes from the outer circumference of the lower roll body 541. The multiple lower convex portions 542 are arranged side by side in an axial direction of the lower roll body 541. Each of the lower convex portions 542 has a tapered shape (in the drawings, approximately a triangular shape) in a cross section when taken in the axial direction of the lower roll body 541. The lower convex portions 542 include multiple central convex portions 542A and multiple lateral convex portions 542B.

The configurations of the central convex portions 542A and the lateral convex portions 542B are similar to those of the upper convex portions 532 (the central convex portions 532A and the lateral convex portions 532B). For this reason, descriptions of the central convex portions 542A and the lateral convex portions 542B are omitted herein. However, the lower convex portions 542 do not always need to be exactly the same as the upper convex portions 532, for example.

The stretch processor 500 stretches the backsheet continuum 120 under the conditions, for example, that: the preheating roll 510 is at 100°C; the stretch processing mechanism 520 is at 80°C; the length (L) of the upper convex portions 532 fitting with the lower convex portions 542 is 1.6 mm; both a pitch (p1) between multiple upper convex portions 532 and a pitch (p2) between multiple lower convex portions 542 are 2.5 mm (see Figs 6 (a) and 6 (b)).

Thereby, the stretch processor 500 forms the stretched portion 21 in the backsheet continuum 120, and stretches the backsheet continuum 120 to 1.3 times the original length of the backsheet continuum 120 before the stretch processing. Here, the stretch processor 500 can be apparently set to any different stretch rate by appropriately changing the above-mentioned conditions.

In the first embodiment as described above, the stretched portion 21 includes the central stretched portion 21A located in the central portion C1 inside the crotch portion S3, and the lateral stretched portions 21B located in the respective lateral portions C2 within the crotch portion S3; the unstretched portions 22 are provided in portions excluding stretched portion 21 (for example, in at least part of the belly waistline portion S1 and the rear waistline portion S2). According to this embodiment, the stretched portion 21 provided in the entire crotch portion S3 is made stretchable in the width direction W of the absorbent article 1, as shown in Fig. 7. In other words, the crotch portion S3 stretches to be wider in the width direction W of the absorbent article 1 than the belly waistline portion S1 and the rear waistline portion S2. For this reason, when a wearer wears the absorbent article 1, a space (i.e., pocket) is formed between the crotch of the wearer and the absorber as shown in Fig. 8, which in turns allows side leakage to be securely prevented.

Additionally, unlike the conventional technique, a leak proof portion folded in multiple layers to form a pocket P does not need to be provided to prevent side leakage. Instead, the pocket P can be formed by simply providing the side flap portion 50. Accordingly, unlike the case of the leak proof portion, since the side flap portion 50 does not need to be folded in multiple layers, the side flap portion 50 is softer than the leak proof portion. Thus, as compared with the case where the leak proof portion is provided, the absorbent article 1 can exhibit improved followability to the movement of a wearer without giving a sense of discomfort to the wearer wearing the absorbent article 1.

In the first embodiment, the stretched portion 21 is provided in the entire central portion C1 in the longitudinal direction L of the absorbent article 1. With this configuration, the backsheet 20 (the backsheet continuum 120) necessary to manufacture the desired absorbent article 1 can be made smaller in width than in the case where the stretched portion 21 is not provided in the entire central portion C1. For this reason, it is possible to reduce an amount of material for the backsheet 20, and thus to lower the manufacturing cost of the absorbent article 1. In addition, since the backsheet 20 is made thin, the central portion C1 of the absorbent article 1 can be soft and easily fit the wearer.

In the first embodiment, the stretch rate of the lateral stretched portions 21B is higher than that of the central stretched portion 21A. Such stretch rate setting can produce the following advantageous effects. Specifically, the central stretched portion 21A is a portion where the absorber 30 is to be placed, and is not made too thin. Thereby, body fluids discharged from a wearer can be prevented from leaking out from the backsheet 20 (the backsheet 20A). In addition, the lateral stretched portions 21B are made softer than the central stretched portion 21A, and thus exhibit improved followability to the movement of a wearer without giving a sense of discomfort to the wearer. Additionally, the lateral stretched portions 21B are made more stretchable than the central stretched portion 21A. This characteristic feature allows a space (i.e., pocket) to be easily formed between the crotch of the wearer and the absorber, and thus, side leakage to be prevented securely.

In the first embodiment, the stretched portion 21 is stretched to at most 2.5 times the original length of the backsheet 20 before the stretch processing. Note that, when the stretch rate of the stretched portion 21 is set at more than 2.5 times the original length of the backsheet 20 before the stretch processing, the backsheet 20 becomes so thin that the backsheet 20 may suffer from damage such as breakage or perforation.

In the first embodiment, the stretched portion 21 preferably has such transparency that the inside of the absorbent article 1 can be seen from the outside. Thus, the inside of the absorbent article 1 can be seen from the outside, and thus the inside of the absorbent article 1 is visually recognizable. Accordingly, it is possible to easily determine when the absorbent article 1 needs changing (replacement time).

### (Modified Example)

The above-mentioned absorbent article 1 according to the first embodiment may be modified as follows. Here, a description will be mainly given of different portions from the above-mentioned first embodiment while attaching the same or similar reference signs to the same or similar portions as in the absorbent article 1 according to the first embodiment.

### (Modified Example 1)

Firstly, a configuration of an absorbent article 1A according to a modified example 1 will be described with reference to the drawings. Fig. 9 is a cross-sectional view showing a part of the absorbent article 1A according to the modified example 1.

In the above-mentioned first embodiment, the backsheet 20 includes the backsheet 20A and the exterior sheet 20B. By contrast, in the modified example 1, the backsheet 20 located in the central portion C1 includes only the backsheet 20A, as shown in Fig. 9.

Specifically, the backsheet 20 located in each of the lateral portions C2 is formed of the backsheet 20A and the side sheet 60. Note that, the side sheet 60 is provided on a surface of the backsheet 20A on a side opposite to the side where the absorber 30 is provided. For example, the side sheet 60 is formed of the same material as that of the exterior sheet 20B.

In this case, the waist flap portions 40 and the side flap portions 50 may be formed of the backsheet 20A and the side sheet 60 extending toward the outer side of the absorbent article 1 in the width direction W.

In the modified example 1, the backsheet 20 located in the central portion C1 includes only the backsheet 20A. For this reason, only a reduced amount of the material is used for the exterior sheet 20B as compared with the case where the backsheet 20 includes the backsheet 20A and the exterior sheet 20B.

### (Modified Example 2)

Next, a configuration of an absorbent article 1B according to a modified example 2 will be described with reference to the drawings. Fig. 10 is a cross-sectional view showing a part of the absorbent article 1B according to the modified example 2.

In the above-mentioned first embodiment, the backsheet 20 includes the stretched portion 21 and the unstretched portion 22. In other words, the stretched portion 21 is formed in the backsheet 20. By contrast, in the modified example 2, the stretched portion 21 is formed not only in the backsheet 20 (the backsheet 20A and the exterior sheet 20B) but also in the topsheet 10, as shown in Fig. 10.

Specifically, both end portions of the topsheet 10 in the width direction W are not wrapped around the absorber 30 but are joined with the backsheet 20A. The above-mentioned stretched portion 21 is formed on a portion where the topsheet 10 is joined with the backsheet 20A.

Here, the method of forming the stretched portion 21 is as follows. Firstly, the backsheet continuum 120 including the backsheets 20 continuously is stretched at a part in the central portion C1. Secondly, the absorber 30 and the topsheet 10 are mounted in this order on the backsheet continuum 120 after the stretch processing. Thirdly, stretch processing is performed on the topsheet continuum including the topsheets 10 continuously and the backsheet continuum 120 which are stretched together at a part in the lateral portions C2.

In the modified example 2, the stretched portion 21 is formed of the topsheet 10 and the backsheet 20. Accordingly, even when body fluids discharged from a wearer flow out to the stretched portion 21, the topsheet 10 after stretch processing can guide the body fluids again to the absorber 30.

### (Modified Example 3)

Next, a configuration of an absorbent article 1C according to a modified example 3 will be described with reference to the drawings. Fig. 11 is a cross-sectional view showing a part of the absorbent article 1C according to the modified example 3.

In the above-mentioned first embodiment, the side sheets 60 are joined with the exterior sheet 20B of the backsheet 20. By contrast, in the modified example 3, the side sheets 60 are joined with the backsheet 20A of the backsheet 20, as shown in Fig. 11.

Specifically, the side sheet 60 is provided on a surface of the backsheet 20A on the side where the absorber 30 is provided. For example, the side sheet 60 is formed of a material different from that of the backsheet 20. The side sheet 60 is stretched in a portion joined with the backsheet 20A by the stretch processing.

Both end portions of the respective side sheets 60 in the width direction W are each provided with a cord-shaped member 61 such as a rubber that is stretchable in the longitudinal direction L of the absorbent article 1C. The cord-shaped member 61 is provided to extend in the longitudinal direction L of the absorbent article 1C while being stretched in the longitudinal direction L of the absorbent article 1C. The cord-shaped member 61 is sandwiched by the doubled side sheet 60 whose end portion in the width direction W is folded.

In the modified example 3, the side sheet 60 including the cord-shaped member 61 is provided in addition to the side flap portion 50. Accordingly, body fluids discharged from a wearer are blocked by the side sheet 60 and the side flap portion 50 (double), and thereby side leakage can be securely prevented.

### [Second Embodiment]

Hereinafter, a configuration of a backsheet 20 according to a second embodiment will be described with reference to the drawings. Fig. 12 is a plan view showing the backsheet 20 according to the second embodiment. Here, a description will be mainly given of different portions from the backsheet 20 of the above-mentioned first embodiment while attaching the same or similar reference signs to the same or similar portions as in the backsheet 20 of the first embodiment.

In the above-mentioned first embodiment, the lateral stretched portions 21B are provided in the lateral portions C2 within the crotch portion S3. In other words, the stretched portion 21 is provided in the entire crotch portion S3 in the width direction W of the absorbent article 1.

By contrast, as shown in Fig. 12, lateral stretched portions 21B are provided in lateral portions C2 excluding edge portions C3 that include edge portions 20E located on the outmost sides of the backsheet 20 in the width direction W. Note that, the lateral stretched portions 21B do not always need to be provided entirely in the lateral portions C2 excluding the edge portions C3. Instead, the lateral stretched portions 21B may be provided in at least part of the lateral portions C2 within the crotch portion S3.

Similarly, in the above-mentioned first embodiment, the central stretched portion 21A is provided in the central portion C1 inside the crotch portion S3. In other words, the stretched portion 21 is provided in the entire central portion C1 in the longitudinal direction L of the absorbent article 1.

By contrast, as shown in Fig. 12, a central stretched portion 21A may be provided in a belly waistline portion S1 and a rear waistline portion S2 excluding edge portions C4 that include edge portions 20E located on the outmost sides L of the backsheet 20 in the longitudinal direction. Note that, the central stretched portion 21A does not always need to be provided entirely in the belly waistline portion S1 and the rear waistline portion S2 excluding the edge portions C4. Instead, the central stretched portion 21A may be provided in at least part of the central portion C1 within the crotch portion S3.

In the second embodiment as described above, the lateral stretched portions 21B are provided in the lateral portions C2 excluding the edge portions C3, and the central stretched portion 21A is provided in the belly waistline portion S1 and the rear waistline portion S2 excluding the edge portions C4. Having these portions, the backsheet 20 can securely prevent side leakage without giving a sense of discomfort to a wearer when the wearer wears the absorbent article as similar to the first embodiment.

Incidentally, an edge portion of the backsheet continuum 120 whose entire surface is stretched by the stretch processing sometimes curls up during the conveyance. This may cause a crease in the backsheet continuum 120, and thereby a manufacturing defect sometimes occurs in the absorbent article 1.

To address this problem, the stretch processing is not performed on the edge portions C3 or edge portions C4 in the second embodiment. For this reason, the rigidity of the edge portions C3 or the edge portions C4 can be secured more than in the case where the stretch processing is performed on the edge portions C3 or the edge portions C4. Accordingly, a curl or crease is unlikely to occur in the backsheet continuum 120 of the backsheet 20 during the manufacturing of the absorbent article 1. Thus, the backsheet continuum 120 can be conveyed stably, which in turn prevents a manufacturing defect in the absorbent article 1.

### [Third Embodiment]

Hereinafter, a configuration of a backsheet 20' according to a third embodiment will be described briefly. Here, a description will be mainly given of portions different from those of the backsheet 20 according to the above-mentioned first embodiment.

In the above-mentioned first embodiment, the stretch rate of the lateral stretched portions 21B is higher than that of the central stretched portion 21A. By contrast, in the third embodiment, the stretch rate of lateral stretched portions 21B is lower than that of a central stretched portion 21A. In this case, the height of lateral convex portions 532B is lower than that of central convex portions 532A.

In the third embodiment as described above, the stretch rate of the lateral stretched portions 21B is lower than that of the central stretched portion 21A. These stretch rate settings also allow the backsheet 20' to more securely prevent side leakage without giving a sense of discomfort to a wearer wearing the absorbent article, as similar to the first embodiment.

In the third embodiment, the rigidity of the central portion C1 can be secured more than in the case where the stretch rate of the lateral stretched portions 21B is higher than that of the central stretched portion 21A, as similar to the second embodiment. Accordingly, a curl or crease is unlikely to occur in the backsheet continuum 120 of the backsheet 20 during the manufacturing of the absorbent article 1. Thus, the backsheet continuum 120 can be conveyed stably, which in turn prevents a manufacturing defect in the absorbent article 1.

### [Other Embodiments]

As described above, the detailed descriptions of the present invention are disclosed through the embodiments. It should not be understood that the descriptions and drawings which constitute a part of this disclosure limit the present invention. Based on this disclosure, those skilled in the art may easily come up with various alternative embodiments, examples and operation techniques.

For example, the embodiments can be modified as follows. Specifically, the absorbent article 1 has been described as an open-type diaper; however, the absorbent article 1 is not limited to this. Instead, the absorbent article 1 may be a pants-type diaper or a sanitary napkin.

In addition, the topsheet 10, the backsheet 20 and the absorber 30 are not limited to the configurations described in the embodiments, but may have a different configuration which can be appropriately modified according to the purpose. Likewise, the configuration of the waist flap portions 40, the side flap portions 50 or the like may also be appropriately changed according to the purpose. In other words, apparently, the stretched portion may be provided in any member different from the above, as long as the stretched portion is provided at least in the backsheet 20.

The lateral stretched portions 21B need to be located in at least part of the lateral portions C2 within the crotch portion S3. Beyond the crotch portion 3, the lateral stretched portions 21B may be provided to partly protrude into the belly waistline portion S1 and the rear waistline portion S2, for example.

In addition, the stretch processor 500 is described as an example to form the stretched portion 21 in the backsheet 20. Accordingly, of course, the stretch processor 500 may have another configuration as long as the configuration is capable of forming the stretched portion 21 in the backsheet 20. In other words, it is obvious that the stretch processor 500 does not always need to include the preheating roll 510 and the stretch processing mechanism 520, and may include only the processing system 520.

As described above, the present invention naturally includes various embodiments which are not described herein. Accordingly, the technical scope of the present invention should be determined only by the matters to define the invention in the scope of claims regarded as appropriate based on the description.

Note that, the entire content of Japanese Patent Application No. 2009-076909 (filed on March 26, 2009) is incorporated herein by reference.

### [Industrial Applicability]

According to the feature of the present invention, it is possible to provide an absorbent article, which can prevent feeling of discomfort when wearing the absorbent article and more effectively prevent a side leakage.

## Claims

1. An absorbent article comprising:
a liquid-permeable topsheet;
a liquid-impermeable backsheet;
an absorber provided between the topsheet and the backsheet;
a belly waistline portion to be fitted to a belly waistline of a wearer;
a rear waistline portion to be fitted to a rear waistline of the wearer;
a crotch portion to be fitted to a crotch of the wearer;
a central portion including the absorber in a width direction of the absorbent article; and
a lateral portion located outside the central portion in the width direction of the absorbent article, wherein
the backsheet includes
a stretched portion stretched by stretch processing in the width direction of the absorbent article perpendicular to a belly-to-rear direction extending from the belly waistline portion to the rear waistline portion, and
a unstretched portion not stretched by the stretch processing, and
the stretched portion includes
a central stretched portion located in at least part of the central portion within the crotch portion, and
a lateral stretched portion located in at least part of the lateral portion within the crotch portion.

2. The absorbent article according to claim 1, wherein
the stretched portion is provided in the entire central portion in the belly-to-rear direction of the absorbent article.

3. The absorbent article according to claim 1, wherein
a stretch rate of the lateral stretched portion is higher than a stretch rate of the central stretched portion.

4. The absorbent article according to claim 1, wherein
a stretch rate of the lateral stretched portion is lower than a stretch rate of the central stretched portion.

5. The absorbent article according to claim 1, wherein
the stretched portion is stretched to at most 2.5 times an original length of the backsheet before the stretch processing.
